# EUROPÄISCHE PATENTSCHRIFT

(11) **EP 1 286 987 B1**
(45) Veröffentlichungstag und Bekanntmachung des Hinweises auf die Patenterteilung: **28.09.2005**
(21) Anmeldenummer: 01942856.4
(22) Anmeldetag: 05.06.2001
(51) Int. Cl.: C07D 403/12, C07D 401/12, C07D 413/12, A61K 31/44, A61P 35/00

(54) **HETEROCYCLISCHE HYDRAZONE ALS ANTI-KREBS-WIRKSTOFFE**
HETEROCYCLIC HYDRAZONES FOR USE AS ANTI-CANCER AGENTS
HYDRAZONES HETEROCYCLIQUES COMME AGENTS ANTI-CANCEREUX

(30) Priorität: 05.06.2000 AT 9772000
(43) Veröffentlichungstag der Anmeldung: 05.03.2003
(60) Teilanmeldung: 03018096.2
(73) Patentinhaber: Austria Wirtschaftsservice Gesellschaft mit beschränkter Haftung, 1030 Wien (AT); Hofmann, Johann, 6020 Innsbruck (AT); Heinisch, Gottfried, 6020 Innsbruck (AT); Easmon, Johnny, 6020 Innsbruck (AT); Pürstinger, Gerhard, 6020 Innsbruck (AT); Fiebig, Heinz Herbert, 79110 Freiburg (DE)
(72) Erfinder: HOFMANN, J., Institut für Med. Univ. Innsbruck, A-6020 Innsbruck (AT); HEINISCH, Gottfried, Institut für Pharmazie, A-6020 Innsbruck (AT); EASMON, Johnny, Institut für Pharmazie, A-6020 Innsbruck (AT); PÜRSTINGER, Gerhard, Institut für Pharmazie, A-6020 Innsbruck (AT); FIEBIG, Heinz-Herbert, Oncotest GmbH, 79108 Freiburg (DE)
(74) Vertreter: Pawloy, Peter Michael
(86) Internationale Anmeldenummer: PCT/AT2001/000187
(87) Internationale Veröffentlichungsnummer: WO 2001/094340

(56) Entgegenhaltungen:
- EASMON, J. ET AL: "Thiazolyl and benzothiazolyl hydrazones derived from.alpha.-(N)- acetylpyridines and diazines: synthesis, antiproliferative activity and CoMFA studies" EUR. J. MED. CHEM. (1997), 32(5), 397-408 , 1997, XP004085514 in der Anmeldung erwähnt
- HALL, IRIS H. ET AL: "Investigations on the mechanism of action of the novel antitumor agents 2-benzothiazolyl, 2-benzoxazolyl, and 2-benzimidazolyl hydrazones derived from 2-acetylpyridine" ARCH. PHARM. (WEINHEIM, GER.) (1999), 332(4), 115-123 , 1999, XP001013551

## Beschreibung

Die vorliegende Erfindung betrifft neue 2-Benzoxazolylhydrazone, die von 2-Formylpyridin, 2-Acylpyridinen, Acetyldiazinen und Acetyl(iso)chinolinen abstammen, ein neues Verfahren zur Herstellung von 2-Benzoxazolylhydrazonen sowie deren Einsatz als nützliche Antikrebs-Therapeutika. Weiters sind diese Verbindungen auch aktiv gegen Krebszellen, die Multidrug-Resistenz zeigen.

Trotz neuen Erkenntnissen in der Tumorbiologie spielen weiterhin chirurgische Eingriffe, Bestrahlung und antitumoral wirksame Substanzen die wichtigsten Rollen in der Tumortherapie. Nachteile der bisher zur Verfügung stehenden Antitumorsubstanzen sind starke Nebenwirkungen, geringe Ansprechraten bei soliden Tumoren und die Entwicklung von Resistenzen. Insbesondere bei Colonkarzinomen, einem der häufigsten Tumore in der westlichen Welt, zeigt die Chemotherapie nur geringe Wirksamkeit. Deshalb wären wirksamere Antitumorsubstanzen wünschenswert.

Das Enzym Ribonukleotid Reduktase (RR) stellt ein wichtiges Zielmolekül in der Chemotherapie von Krebs dar. In der Absicht eine neue Klasse von RR-Inhibitoren zu entwickeln, wurde das N-N-S-Pharmacophor von α-(N)-heteroaromatischen Thiosemicarbazonen, z.B. Verbindungen (1) und (2), als Ausgangspunkt gewählt.

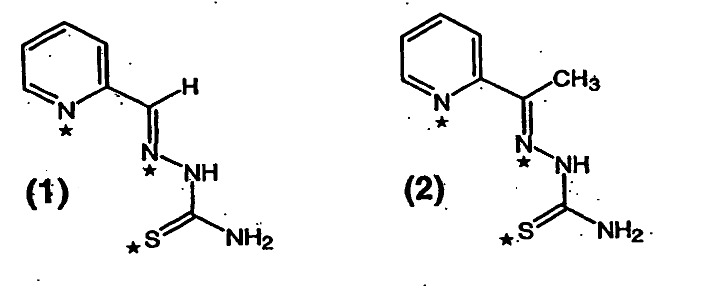

Weiters wurden 2-Benzothiazolyl- und 2-Thiazolylhydrazone, die von 2-Formylpyridin, z.B. Verbindungen (3) und (4), 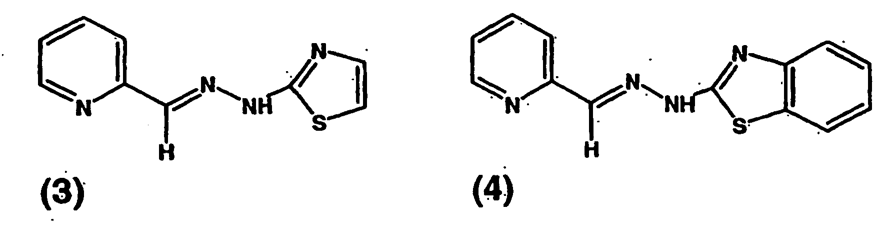 beziehungsweise 2-Acetylpyridinen, z.B. Verbindungen (5) und (6) 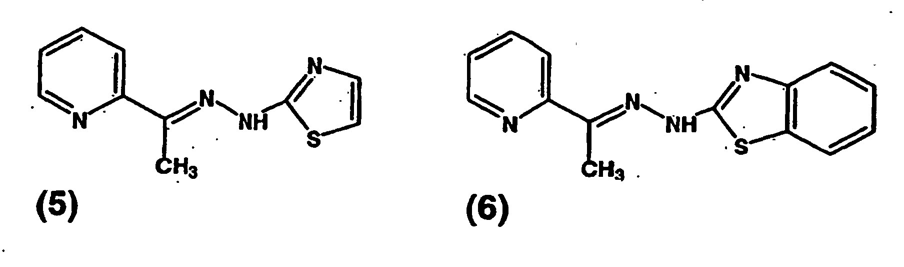 abstammen, bereits synthetisiert.

Diese Verbindungen wurden auch bereits *in vitro* gegen eine Reihe von humanen Tumor-Zellinien getestet, wobei die bekannten Verbindungen (1) und (2) als Kontrollen dienten. Die Hydrazone (3) und (5) stellten sich dabei als 5 bis 10 mal aktiver als die bekannten Thiosemicarbazone (1) und (2) heraus (siehe *Easmon et al., Eur. J. Med. Chem., 32, 397 (1997)*). Weiters konnte auch bereits gezeigt werden, dass die Verbindungen (3) bis (6) keine Krenzresistenz gegen eine Leukämie-Zellinie aufweisen, welche die M2 Proteinuntereinheit überexprimiert. Jedoch stellte sich dabei auch heraus, dass die Verbindungen (3) bis (6) die RR nicht hemmen und dass das N-N-S Pharmakophor für diese Klasse von Wirkstoffen nicht relevant ist. Diese Vermutung wurde auch dadurch unterstützt, dass diese Verbindungen Metallionen in der N-N-N Form binden, wie die Röntgenstrukturanalyse des Nickelkomplexes der Verbindung (6) ergab.

In der Weiterführung der Bemühungen, neue Antitumor-Wirkstoffe zu entwickeln, wurden nun gemäß der vorliegenden Erfindung Hydrazone dargestellt, bei denen das 2-Benzothiazolylringsystem durch ein 2-Benzoxazolylringsystem ersetzt wurde. Das führte zu einer neuen Klasse von Hydrazonen, die potente cytotoxische und antitumorale Aktivität zeigt und auch gegen Multidrug-resistente Tumore nützlich ist.

Die antiproliferative Aktivität der neuen Substanzen wurden in verschiedenen humanen Tumorzellinien getestet. Wirksame Verbindungen wurden dann im Clonogenic Assay (Hemmung der Koloniebildung von humanen Tumortransplantaten in Softagar) untersucht.

In allen Versuchen zeigten die Substanzen Antitumoraktivität, insbesondere gegen Colontumoren.

Bei der intensiven Suche nach Verbindungen mit Antitumoraktivität wurde nun überraschenderweise gefunden, dass neue Hydrazone, abgeleitet von (benzoannellierten) α-(*N*)-Formyl- und Acyl-(Di)azinen und 2-Hydrazinobenzoxazolen beachtliche Antitumoraktivität sowohl *in vitro* als auch *in vivo* zeigen.

Die vorliegende Erfindung betrifft nun neue Verbindungen der allgemeinen Formel 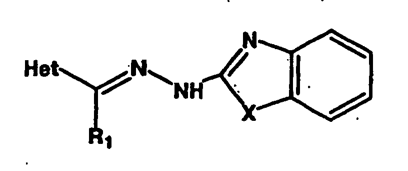 wobei Het. = 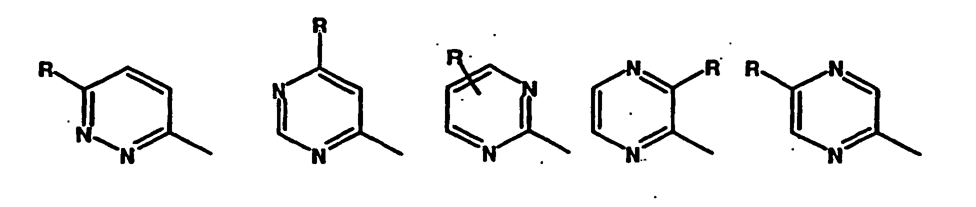 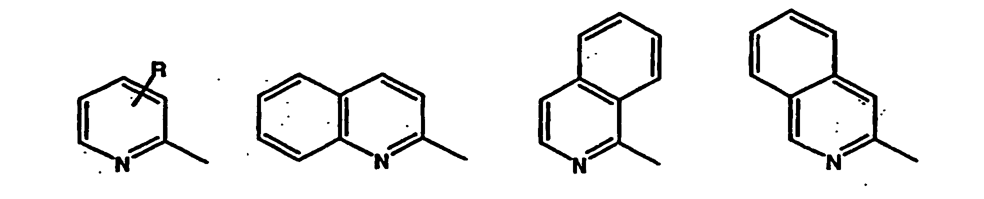 und wobei R = H, CH₃, OCH₃, OH, Cl, Br, F, CF₃, NO₂, NH₂, NHCOCH₃, N(CH₃)₂, Phenyl, CN, C=NH(NH₂), C=S(NH₂), C=NH(NHOH), COOH oder COOR₄, wobei R₄ = aliphatischer Rest oder Phenylgruppe, oder CONR₅R₆, wobei R₅ und R₆H, einen aliphatischen Substituenten oder eine Phenylgruppe bedeuten,
R₁ = H, Methyl, Ethyl, Propyl, *iso*-Propyl, Butyl, *tert*.-Butyl, Cyclopropyl, Cyclohexyl, Phenyl, Benzyl, oder 2-Pyridyl, und
X = O ist,
   mit der Maßgabe, dass, wenn Het = , 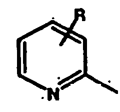 wobei R = H,
R₁ nicht Methyl bedeutet,
sowie deren pharmazeutisch annehmbaren Salze.

Die vorliegende Erfindung betrifft ebenfalls ein Verfahren zur Herstellung einer Verbindung der allgemeinen Formel 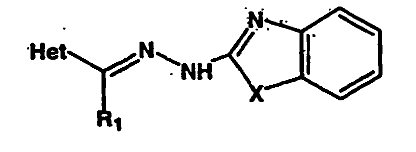 wobei Het. = 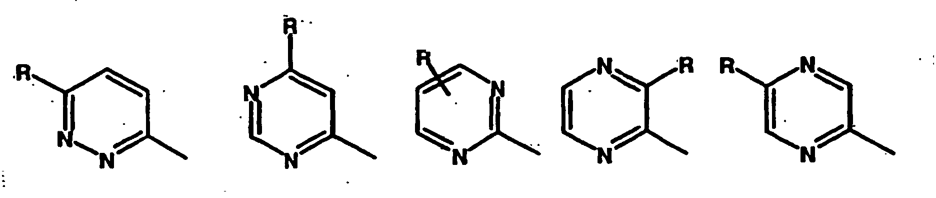 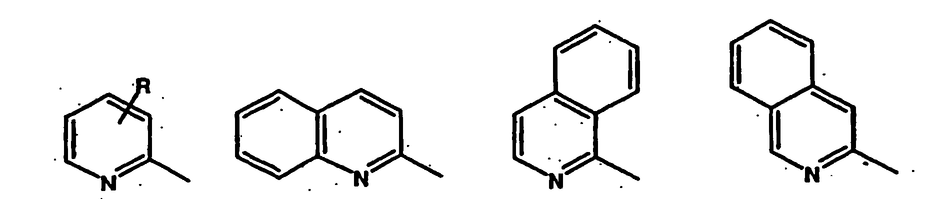 und wobei R = H, CH₃, OCH₃, OH, Cl, Br, F, CF₃, NO₂, NH₂, NHCOCH₃, N(CH₃)₂, Phenyl, CN, C=NH(NH₂), C=S(NH₂), C=NH(NHOH), COOH oder COOR₄, wobei R₄ = aliphatischer Rest oder Phenylgruppe, oder CONR₅R₆, wobei R₅ und R₆ H, einen aliphatischen Substituenten oder eine Phenylgruppe bedeuten,
R₁ = H, Methyl, Ethyl, Propyl, *iso*-Propyl, Butyl, *tert*.-Butyl, Cyclopropyl, Cyclohexyl, Phenyl, Benzyl, oder 2-Pyridyl, und X = O bedeutet, durch Umsetzen geeigneter Ketone mit passend substituierten Hydrazinen. Insbesondere können die obgenannten Verbindungen sowie geeignete Zwischenverbindungen durch das folgende Verfahren hergestellt werden: 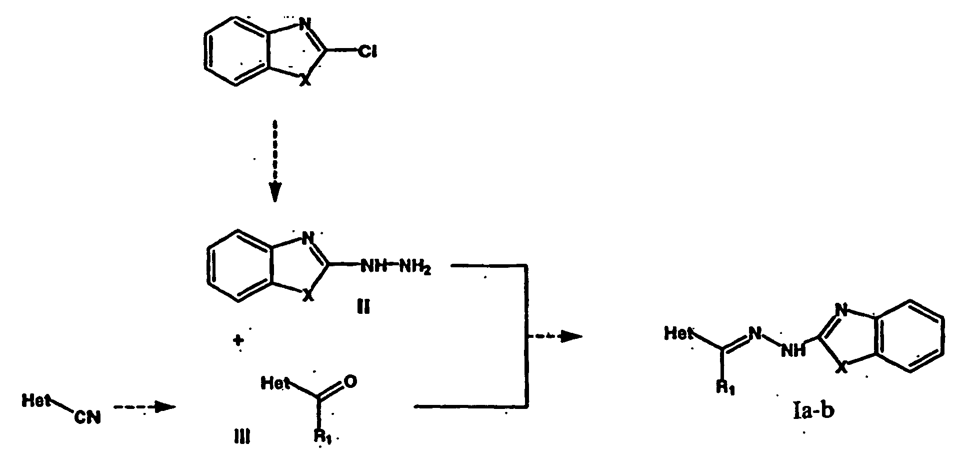
wobei Het, R, R₁, R₂, R₃, R₄, R₅, R₆ und X wie oben definiert sind.

Die Hydrazone des Typs Ia-b werden durch Erhitzen eines Ketons (III) und eines Hydrazins (II) in Methanol oder Ethanol unter Zugabe einer katalytischen Menge einer geeigneten Säure, wie z.B. Essigsäure, Salzsäure oder Schwefelsäure, dargestellt Die Synthese kann alternativerweise auch bei Raumtemperatur durchgeführt werden, dauert dann aber mehrere Tage bis zum vollständigen Umsatz. Die Hydrazine des Typs II werden durch Erhitzen der entsprechenden 2-Chlor-Edukte mit 98%igem Hydrazinhydrat auf Rückflusstemperatur nach Standardvorschriften hergestellt, beispielsweise siehe *Katz. L., J Am Chem Soc 75, 712, (1953).*

Die Ketone des Typs III wurden durch Umsatz von entsprechenden 2-Cyano-verbindungen mit einem geeigneten Grignardreagens (RMgX), Alkyllithium- oder Phenyllithiumreagens - in Analogie zu bekannten Verfahren bzw. zur Patentliteratur (siehe *Lutz, H. et at. DE-43 06 006-A*) - synthetisiert.

### Pharmakologische Tests:

Die überraschende Antitumor-Aktivitäten der neuen bzw. bereits im Stand der Technik allgemein geoffenbarten Hydrazone der vorliegenden Erfindung werden im folgenden beschrieben. Als Kontrolle wird Hydroxyharnstoff, ein kommerziell erhältliches Krebs-Chemotherapeutikum, herangezogen.

### Hemmung desTumorzellwachstums.

Um Information über die wachstumshemmende Wirkung auf Tumorzellen zu bekommen, wurde die Hemmung des Wachstums folgender humaner Tumorzellen bestimmt: Burkitt's Lymphoma (CA 46, ATCC Nr. CRL 1648), CCRF-CEM (akute lymphoblastische Leukämie, ATCC Nr. CCL 119), K562 (chronisch myeloische Leukämie, ATCC Nr. CCL 243), HeLa (epitheloides Cervix Karzinom, ATCC Nr. CCL 2), MEXF 276L (Melanom), HT-29 (Colon Adenokarzinom, ATCC Nr. HTB 38), KB-3-1 (humanes Oral-Epidermoid Karzinom, CTCC Nr. CCL 17), KB-HU Hydroxyharnstoff resistente, Multidrug resistente KB-C1 Zellen *(Akiyama et al., Cell. Mol. Genet., 11:117-126, 1985*). Burkitt's, CCRF-CEM, HeLa und MEXF 276L Zellen wurden in RPMI 1640, HT-29 Zellen in McCoy's 5A Medium gezogen. Die KB Zellinien wurden in Dulbecco's modified Eagle's medium (4,5 g Glucose/1) gezogen. Zu den KB-C1 Kulturen wurde jede 2. Woche 1µg Colchizin/ml, zu den Hydroxyharnstoff-resistenten KB-HU Zellen 1 mM Hydroxyharnstoff zugegeben. Die Medien wurden mit 10 % fötalem Kälberserum (ausgenommen Burkitt's Lymphom Zellen mit 15 %), 2 mM Glutamin, 50 units/ml Penicillin and 50 µg/ml Streptomycin supplementiert. Die Wachstumshemmung von HeLa, HT-29, KB und MEXF 276L Zellen wurde mit dem SRB-Assay bestimmt *(Skehan et al., J. Natl. Cancer. Inst., 82:1107-1112, 1990*). 3 000 - 10 000 Zellen wurden in 200 µl Medium pro Well in 96-Well Platten ausplattiert. Die Dosis-Wirkkurven für CCRF-CEM und Burkitt's Lymphom Zellen wurden mit dem MTT-Assay (*Mosman, J. Immunol. Methods, 65:55-63, 1983*) von Boehringer Mannheim, Mannheim, Deutschland, erstellt. Dazu wurden ca. 10 000 Zellen pro 100 µl in 96-Well Platten ausgesät. Nach einer anfänglichen Inkubation über vier Stunden wurden verschiedene Substanzkonzentrationen zugegeben und die Zellen 72 Stunden bei 37° C in wassergesättigter 95%iger Luft und 5%igem CO₂ inkubiert. Die Substanzen wurden in Dimethylsulfoxid (DMSO) gelöst. Die DMSO-Konzentration war 0,5 % und diese war für die Zellen nicht toxisch. Danach wurden die Proben fixiert, gewaschen und die Absorption in einem Microplate Reader bestimmt.

Die Ergebnisse werden in den Tabellen 1 und 2 wiedergegeben

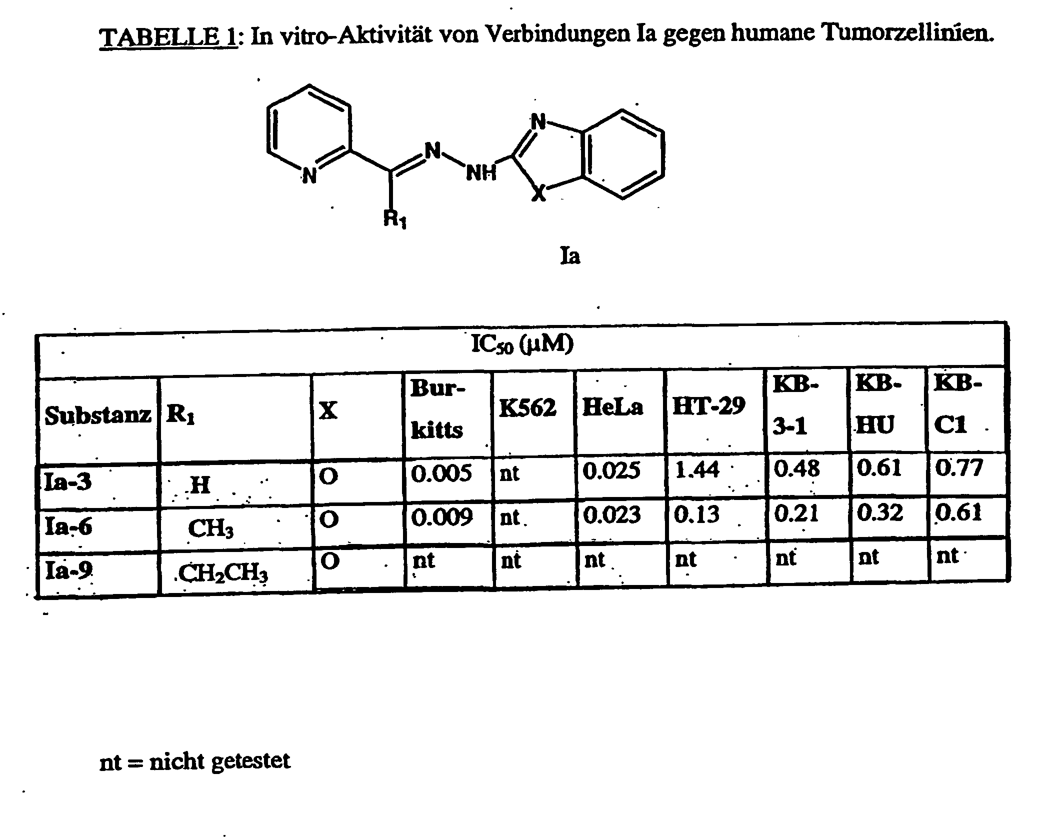

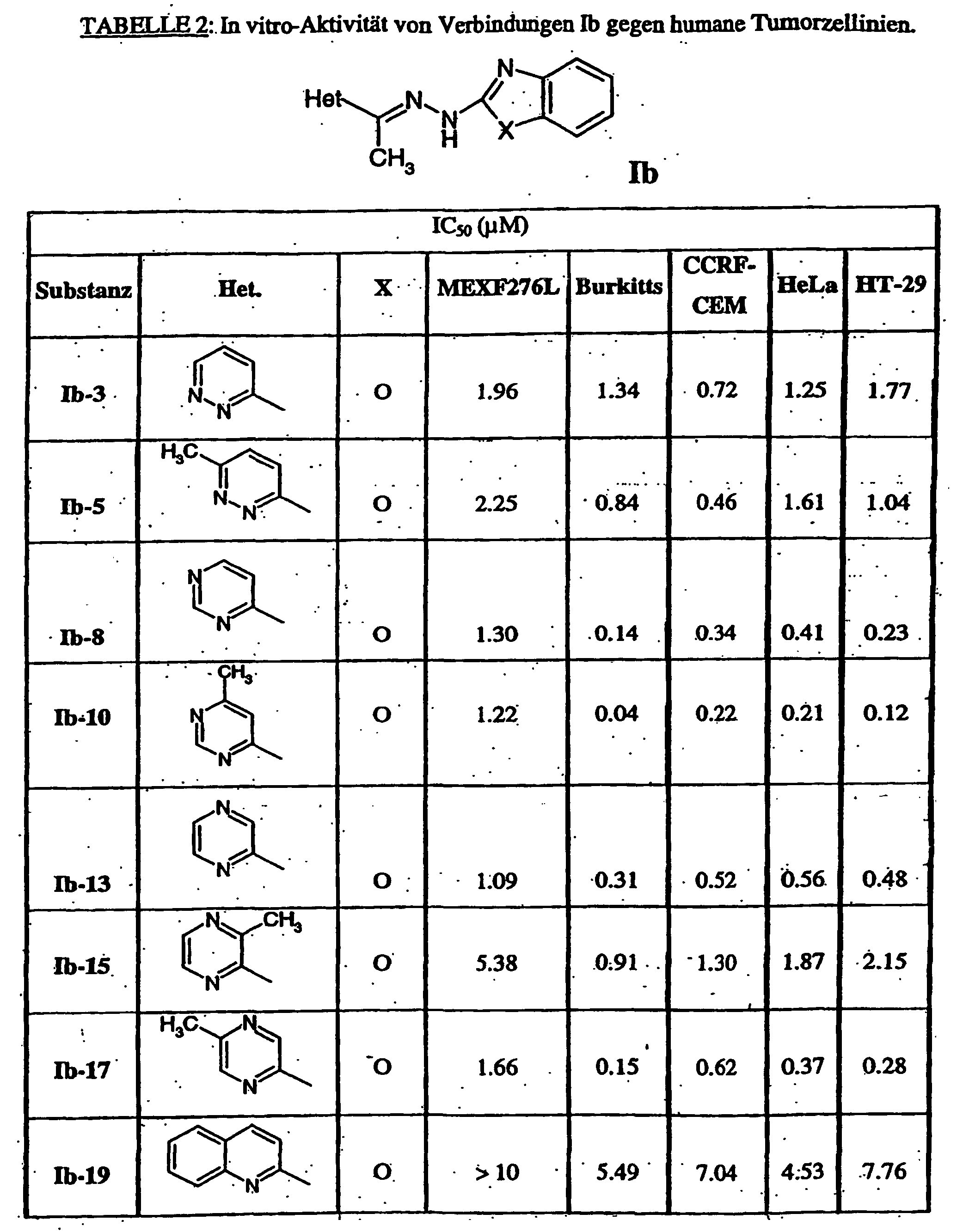

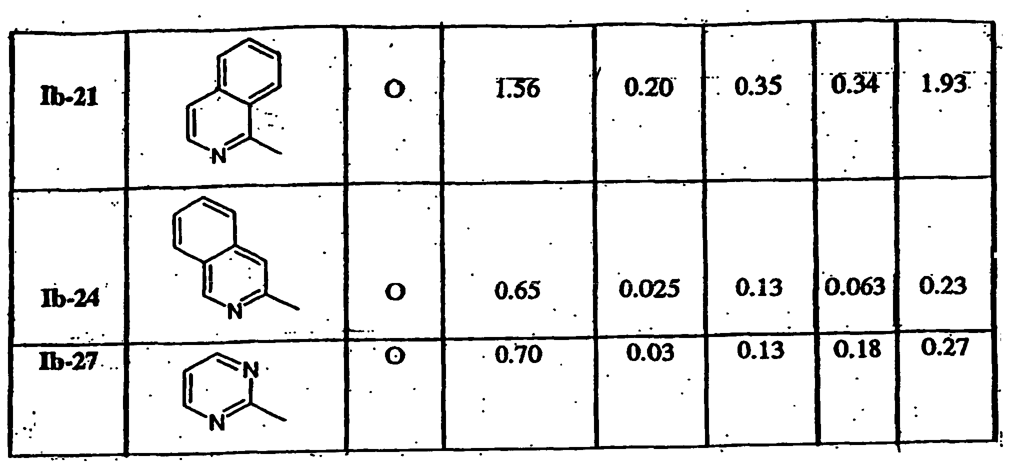

### Kolonienbildungsassay als genauere in vitro-Untersuchung

Um detaillierte Informationen darüber zu erhalten, bei welchem Tumortyp die Substanzen die beste Wachstumshemmung bewirken, wurde die Koloniebildung von humanen Tumortransplantaten getestet. Zwischen der Ansprechrate in Patienten und dem Koloniebildungsassay wurde eine ausgezeichnete Korrelation gefunden *(Scholz et al., Eur. J. Cancer 25:901-905, 1990*). Solide humane Tumoren wurden als Transplantate in Nacktmäusen gezogen, aus diesen entfernt, mechanisch zerkleinert und danach bei 37°C für 30 Minuten in einem Enzymcocktail inkubiert, der aus Kollagenase (1,2-1,8 U/mL), DNAse (375 U/mL) und Hyaluronidase (29 U/mL) in RPMI 1640 Medium bestand. Die Zellmischung wurde durch Siebe mit 200 µm und 50 µm Porengröße passiert und danach zweimal mit PBS (phosphate buffered saline) gewaschen. Der Prozentsatz von lebenden Zellen wurde mit einer Neubauerzählkammer und Trypanblaufärbung gezählt.

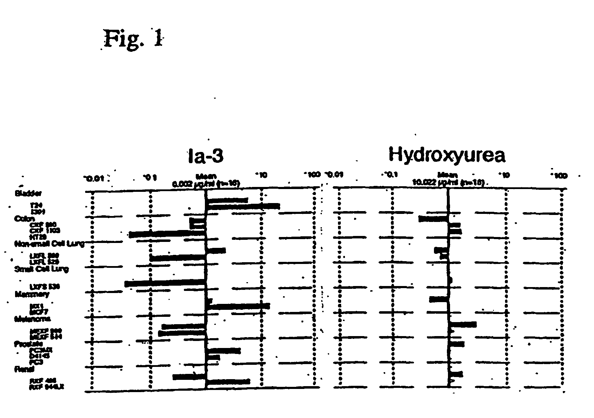

Der Koloniebildungsassay wurde mit einer von Hamburger und Salmon *(Hamburger and Salmon, Science, 197: 461-463, 1977)* eingeführten und modifizierten Zweischichtagartechnik durchgeführt. Die untere Schicht bestand aus 0,2 ml Iscove's modified Dulbecco's Medium mit 20% fötalem Kälberserum und 0,75% Agar. 8x10³ bis 1.6x10⁴ Zellen wurden in dasselbe Medium und 0,4% Agar gegeben und in 24-Multiwell Platten auf die untere Schicht plattiert. Die Substanzen wurden einen Tag nach dem Ausplattieren (bei andauernder Exposition) in 0,2 ml Medium zugegeben. Jede Platte enthielt 6 Kontrollen nur mit dem Lösungsmittel und die behandelten enthielten je dreifach 6 Konzentrationen der Substanzen. Die Kulturen wurden, je nach Verdoppelungszeit der Tumorzellen, 3 bis 6 Tage bei 37 °C und 7% CO₂ in wassergesättigter Atmosphäre inkubiert. Zum Zeitpunkt der maximalen Koloniebildung mit einer Größe von 50 µM wurden diese mit einem automatischen Imageanalysesystem ausgezählt. 24 Stunden vor Auszählung wurden die lebenden Kolonien mit einer sterilen wäßrigen Lösung von 2-(4-Iodophenyl)-3-(4-nitrophenyl)-5-phenyltetrazolium chloride (1mg/ml, 100µl/Well) gefärbt. Die Ergebnisse der Tests werden in Fig. 1 gezeigt. In Fig. 1 zeigen Säulen nach links, dass die betreffenden Zelllinien sensitiver sind als der Durchschnitt. Säulen nach rechts zeigen geringere Aktivität als der Durchschnitt. Die folgenden Zelllinien wurden untersucht:

| Tumor | Zelllinie | Histologie | Kontaktzeit mit Testverbindung (Tage) |
|---|---|---|---|
| Blase | T24 | | |
| | | | |
| Brust | MAXF 401NL | Adenocarcinom ER-, Pr- | 6 |
| | MCF-7 | Adenocarcinom ER+, Pr+ | 4 |
| | MDA-MB 468 | | |
| | | | |
| Dickdarm | HT-29 | mäßig diff. Adenocarcinom | 3 |
| | SW620 | schwach diff. Adenocarcinom | 3 |
| | | | |
| Dickdarm | CXF 94L | | |
| | | | |
| Magen | GXF 251L | Adenocarcinom | 4 |
| | | | |
| Lunge - small cell | DMS 114 | | |
| | DMS 273 | | |
| | | | |
| Lunge - non small cell | LXFA 526L | Adenocarcinom | |
| | LXFA 629L | Adenocarcinom | 4 |
| | LXFE 66L | Epidermoid Carcinom | 4 |
| | LXFL 529L | large cell Carcinom | |
| | LXFL 1072L | large cell Carcinom | |
| | | | |
| Melanom | MEXF 462NL | amelanoides Melanom | 4 |
| | MEXF 514NL | melanoides Melanom | 4 |
| | | | |
| Ovarial | OVCAR3 | Adenocarcinom | 6 |
| | OVXF 899L | | |
| | | | |
| Prostata | DU145 | | |
| | PC3M | | |
| | | | |
| Renal | RXF 486L | Hypernephrom | 4 |
| | RXF 944L | Hypernephrom | 4 |
| | | | |
| Gebärmutter | UXF 1138L | Carcinosarcom | 4 |
| ER = Estrogen Rezeptor, Pr = Progesteron Rezeptor, (-) = negativ, (+) = positiv | | | |

Wie ersichtlich, zeigen die Verbindungen der vorliegenden Erfindung ausgezeichnete *in vitro* Antitumoraktivitäten (IC₅₀) gegen humane Krebszellen. Im Vergleich ist die Aktivität von Hydroxyharnstoff viel geringer als die der erfindungsgemäßen Verbindungen (vgl. Fig. 1). Das IC₇₀-Muster der Substanzen ist sehr ähnlich, woraus geschlossen werden kann, dass sie den identischen Wirkmechanismus haben. Weiters zeigt die Verbindung **Ia-24** Selektivität für Dickdarm-, Brust-, Ovarial- und Gebärmutterkrebs.

Die Substanz **Ib-24** wurde auch im sog. "Hollow Fibre Assay" auf ihre Antitumoraktivität getestet. Dabei werden Mäusen bis zu 12 verschiedene Tumorzellen in durchlässigen Schläuchen implantiert und mit den erfindungsgemäßen Verbindungen behandelt. Bei diesen Versuchen wurde bestätigt, dass die Substanz **Ib-24** Antitumoraktivität zeigt.

Die Herstellung von Verbindungen der vorliegenden Erfindung wird nun anhand der nachfolgenden Beispiele, auf die sie jedoch nicht beschränkt sein soll, erläutert. Die angegebenen NMR-Daten beziehen sich auf Messung in DMSO-d6.

### Beispiel 1:

### 1-(2-Pyridyl)-1-propanon-1-(1,3-benzoxazol-2-yl)hydrazon (Ia-13)

Eine Mischung von 2-Propionylpyridin (0,60 g, 4,42 mmol) und 2-Hydrazinobenzoxazol (0,60 g, 4,02 mmol) in 25 ml Methanol wird nach Zugabe von 6 Tropfen Eisessig 24 Stunden unter Rückfluss erhitzt. Das Reaktionsgemisch über Nacht im Kühlschrank bei ca. 5 °C aufbewahrt. Der Niederschlag wird abfiltriert und aus Diisopropylether umkristallisiert. Ausbeute: 0,68 g (64 % d.Th.).

| C₁₅H₁₄N₄O (266.31) | | | | |
|---|---|---|---|---|
| CHN | Ber. | C 67,65% | H 5,30% | N 21,04% |
| | Gef. | C 67,76% | H 5,28% | N 21,24% |

¹H-NMR (δ, ppm) = 1,08 (t, 3H), 3,05 (q, 2H), 7,05-7,46 (m, 4H), 7,37 (qd, 1H), 7,84 (ddd, 1H), 8,22 (br.s 1H), 8,60 (qd, 1H), 11,48 (br.s, 1H).

### Beispiel 2:

### 1-(2-Pyrazinyl)-1-ethanon-1-(1,3-benzoxazol-2yl)hydrazon (Ib-13)

Eine Mischung von 4-Acetylpyrazin (0,41 g, 3,35 mmol) und 2-Hydrazinobenzoxazol (0,50 g, 3,35 mmol) in 15 ml Methanol wird nach Zugabe von 5 Tropfen Eisessig unter Rückfluss erhitzt, bis die Reaktionskontrolle mittels Dünnschichtchromatographie (Polygrarn Sil G/UV₂₅₄ Fertigfolien; Laufmittel: Petrolether:Ethylacetat (3:7)) keinen weiteren Umsatz anzeigt (ca. 15 Stunden). Das Reaktionsgemisch wird über Nacht im Kühlschrank bei ca. 5 °C aufbewahrt. Der Niederschlag wird abfiltriert und aus Methanol umkristallisiert. Ausbeute: 0,75 g (80 % d.Th.).

| C₁₃H₁₁N₅O (253.27) | | | | |
|---|---|---|---|---|
| CHN | Ber. | C 61,65% | H 4,38% | N 27,65% |
| | Gef. | C 61,82% | H 4,52% | N 28,04% |

¹H-NMR (8, ppm) = 2,38 (s, 3H), 7,04-7,46 (m, 4H), 8,58 (dd, 1H), 8,60 (dd, 1H), 9,47 (br. s, 1H), 11,63 (br. s, 1H).

## Patentansprüche

1. Verbindung der allgemeinen Formel: 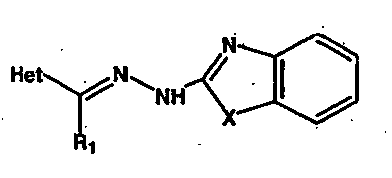 wobei Het. = 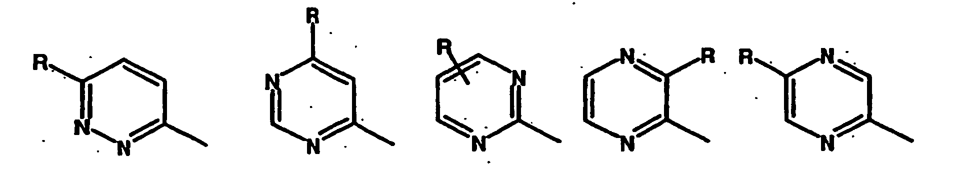 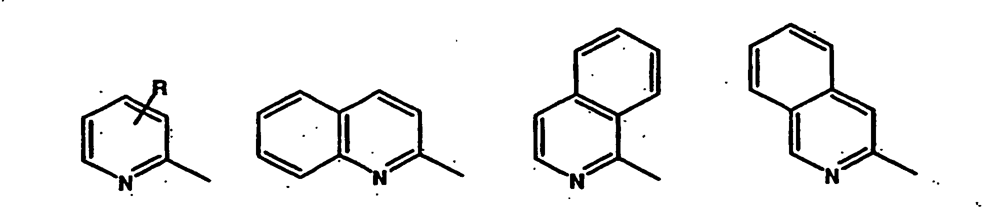 und wobei R = H, CH₃, OCH₃, OH, Cl, Br, F, CF₃, NO₂, NH₂, NHCOCH₃, N(CH₃)₂, Phenyl, CN, C=NH(NH₂), C=S(NH₂), C=NH(NHOH), COOH oder COOR₄, wobei R₄ = aliphatischer Rest oder Phenylgruppe, oder CONR₅R₆, wobei R₅ und R₆ H, einen aliphatischen Substituenten oder eine Phenylgruppe bedeuten,
R₁ = H, Methyl, Ethyl, Propyl, *iso*-Propyl, Butyl, *tert*.-Butyl, Cyclopropyl, Cyclohexyl, Phenyl, Benzyl, oder 2-Pyridyl, und
X = O bedeutet,
mit der Maßgabe, dass, wenn Het = , 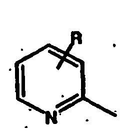 wobei R = H,
R₁ nicht Methyl bedeutet,
sowie deren pharmazeutisch annehmbaren Salze.

2. Verbindung nach Anspruch 1, nämlich 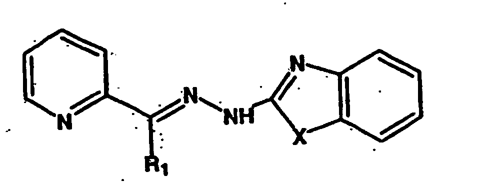 wobei R₁ = H, Methyl, Ethyl, Propyl, *iso*-Propyl, Butyl, *tert*.-Butyl, Cyclopropyl, Cyclohexyl, Phenyl, Benzyl oder 2-Pyridyl und X = O,
mit der Maßgabe, dass,
R₁ nicht Methyl bedeutet,
sowie deren pharmazeutisch annehmbaren Salze.

3. Verbindung nach Anspruch 1, nämlich 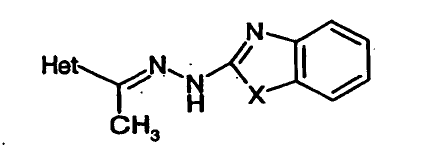 wobei Het. = 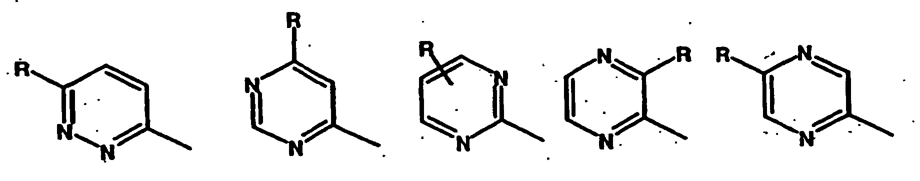 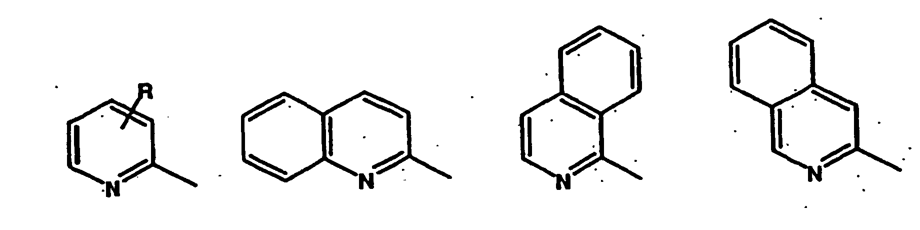 R = H oder CH₃, X = O,
mit der Maßgabe, dass, wenn Het = 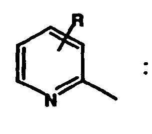 R nicht H bedeutet,
sowie deren pharmazeutisch annehmbaren Salze.

4. Verfahren zur Herstellung einer Verbindung der allgemeinen Formel 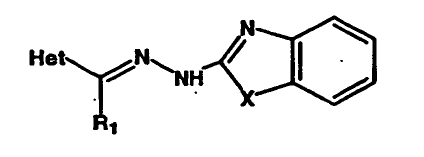 wobei Het. = 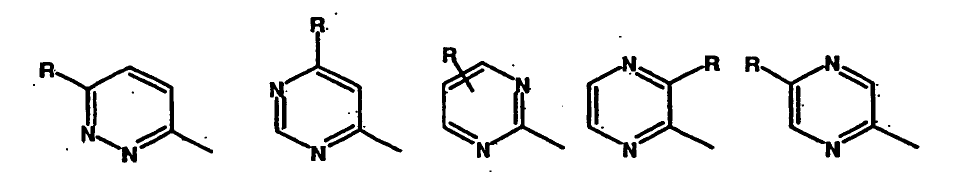 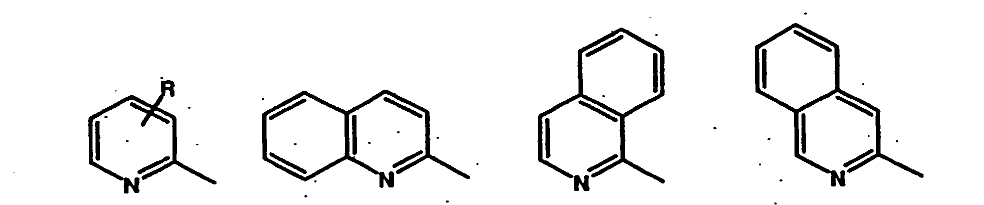 und wobei R = H, CH₃, OCH₃, OH, Cl, Br, F, CF₃, NO₂, NH₂, NHCOCH₃, N(CH₃)₂, Phenyl, CN, C=NH(NH₂), C=S(NH₂), C=NH(NHOH), COOH oder COOR₄, wobei R₄ = aliphatischer Rest oder Phenylgruppe, oder CONR₅R₆, wobei R₅ und R₆ H, einen aliphatischen Substituenten oder eine Phenylgruppe bedeuten,
R₁ = H, Methyl, Ethyl, Propyl, *iso*-Propyl, Butyl, *tert*.-Butyl, Cyclopropyl, Cyclohexyl, Phenyl, Benzyl, oder 2-Pyridyl, und X = O bedeutet, **dadurch gekennzeichnet, dass** ein Keton der allgemeinen Formel (III) 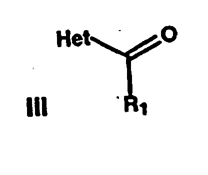
worin Het und R₁ die oben angegebene Bedeutung haben, mit einem Hydrazin der allgemeinen Formel (II) 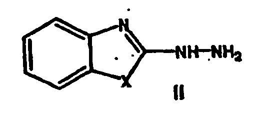 worin X die oben angegebene Bedeutung hat, umgesetzt wird.

5. Verfahren nach Anspruch 4, **dadurch gekennzeichnet, dass** die Umsetzung in Methanol oder Ethanol durchgeführt wird.

6. Verfahren nach Anspruch 4 oder 5, **dadurch gekennzeichnet, dass** die Umsetzung in Gegenwart einer katalytischen Menge einer geeigneten Säure, wie Essigsäure, Salzsäure oder Schwefelsäure, durchgeführt wird.

7. Verbindung der allgemeinen Formel: 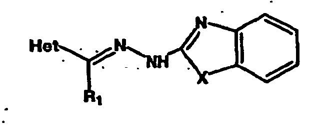 wobei Het. = 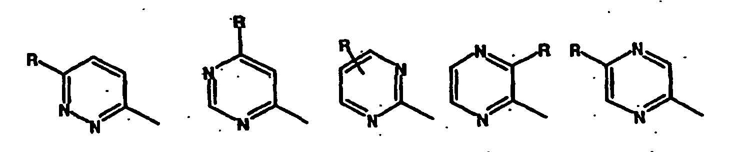 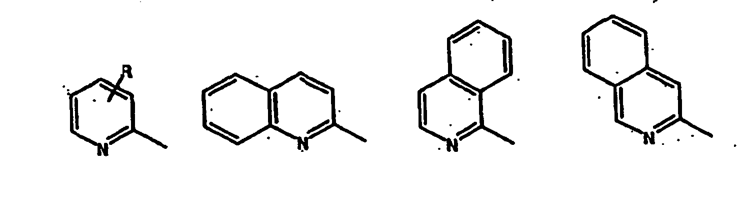 und wobei R = H, CH₃, OCH₃, OH, Cl, Br, F, CF₃, NO₂, NH₂, NHCOCH₃, N(CH₃)₂, Phenyl, CN, C=NH(NH₂), C=S(NH₂), C=NH(NHOH), COOH oder COOR₄, wobei R₄ = aliphatischer Rest oder Phenylgruppe, oder CONR₅R₆, wobei R₅ und R₆ H, einen aliphatischen Substituenten oder eine Phenylgruppe bedeuten,
R₁ = H, Methyl, Ethyl, Propyl, *iso*-Propyl, Butyl, *tert*.-Butyl, Cyclopropyl, Cyclohexyl, Phenyl, Benzyl, oder 2-Pyridyl, und X = O bedeutet, sowie deren pharmazeutisch annehmbaren Salze zur Verwendung als Arzneimittel.

8. Verbindung wie in Anspruch 7 definiert zur Verwendung als Antikrebs-Therapeutikum.

9. Verbindung wie in Anspruch 7 definiert zur Verwendung als Antitumor-Therapeutikum.

10. Verwendung einer Verbindung wie in Anspruch 7 definiert bei der Herstellung eines Medikaments zur Behandlung von Dickdarm-, Brust-, Ovarial- oder Gebärmutterkrebs.

## Claims

1. A compound of the general formula 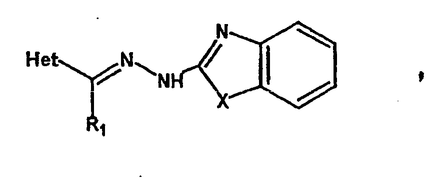 wherein Het = 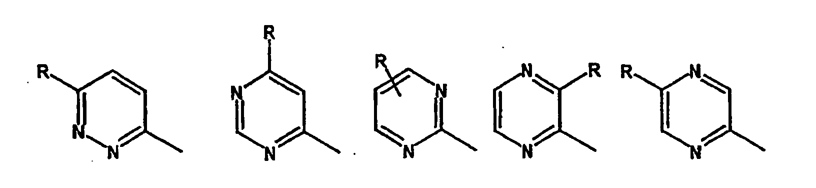 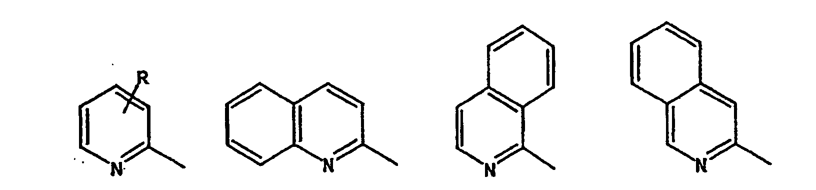 and wherein R = H, CH₃, OCH₃, OH, Cl, Br, F, CF₃, NO₂, NH₂, NHCOCH₃, N(CH₃)₂, phenyl, CN, C=NH(NH₂), C=S(NH₂), C=NH (NHOH), COOH or COOR₄, wherein R₄ = an aliphatic residue or a phenyl group, or CONR₅R₆, wherein R₅ and R₆ are H, an aliphatic substituent or a phenyl group,
R₁ = H, methyl, ethyl, propyl, *iso*-propyl, butyl, *tert*.-butyl, cyclopropyl, cyclohexyl, phenyl, benzyl or 2-pyridyl, and
X = O,
with the proviso that if Het= 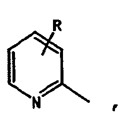 wherein R = H,
R₁ is not methyl,
as well as the pharmaceutically acceptable salts thereof.

2. A compound according to claim 1, namely 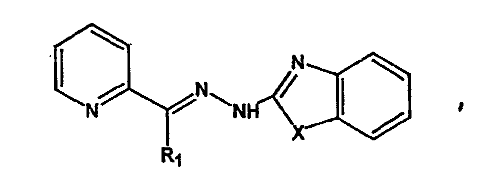 wherein R₁ = H, methyl, ethyl, propyl, *iso*-propyl, butyl, *tert*.-butyl, cyclopropyl, cyclohexyl, phenyl, benzyl or 2-pyridyl, and X = O,
with the proviso that
R₁ is not methyl,
as well as the pharmaceutically acceptable salts thereof.

3. A compound according to claim 1, namely 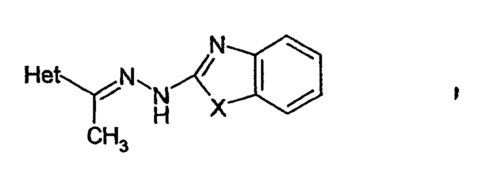 wherein Het = 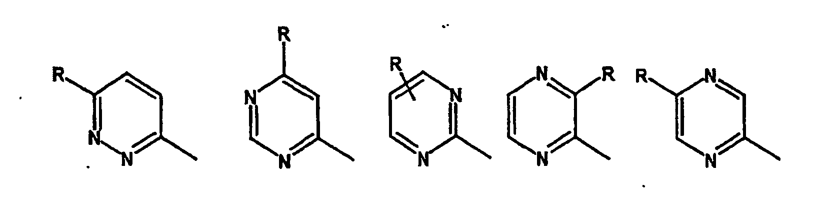 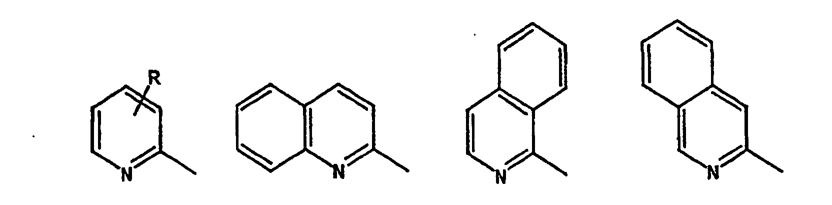
R = H or CH₃, X = O, with the proviso that if Het = 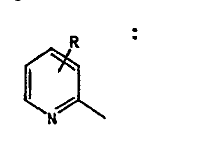
R is not H,
as well as the pharmaceutically acceptable salts thereof.

4. A method for preparing a compound of the general formula 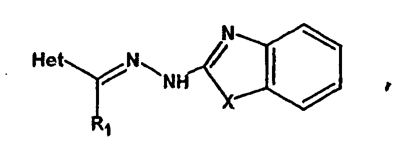 wherein Het = 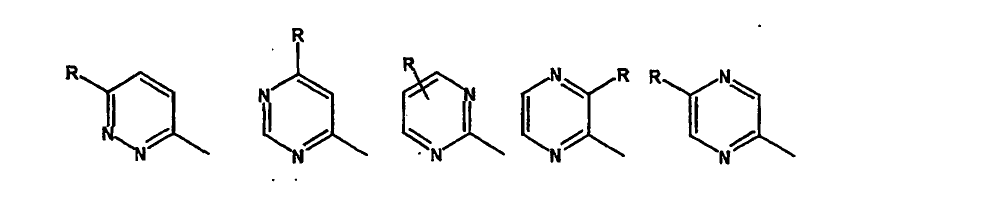 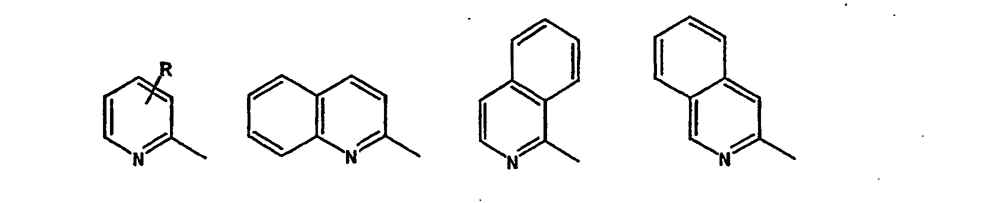 and wherein R = H, CH₃, OCH₃, OH, Cl, Br, F, CF₃, NO₂, NH₂, NHCOCH₃, N(CH₃)₂, phenyl, CN, C=NH(NH₂), C=S(NH₂), C=NH(NHOH), COOH or COOR₄, wherein R₄ = an aliphatic residue or a phenyl group, or CONR₅R₆, wherein R₅ and R₆ are H, an aliphatic substituent or a phenyl group,
R₁ = H, methyl, ethyl, propyl, *iso*-propyl, butyl, *tert*.-butyl, cyclopropyl, cyclohexyl, phenyl, benzyl, or 2-pyridyl, and X = O, **characterised in that** a ketone of the general formula (III) 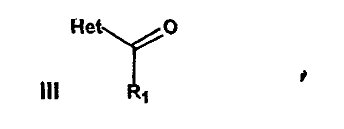
wherein Het and R₁ are as defined above, is reacted with a hydrazine of the general formula (II) 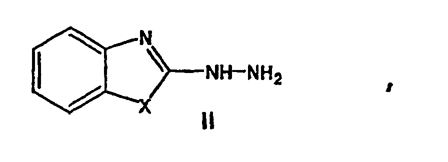 wherein X is as defined above.

5. A method according to claim 4, **characterised in that** the reaction is carried out in methanol or ethanol.

6. A method according to claim 4 or 5, **characterised in that** the reaction is carried out in the presence of a catalytic amount of a suitable acid, such as acetic acid, hydrochloric acid or sulphuric acid.

7. A compound of the general formula: 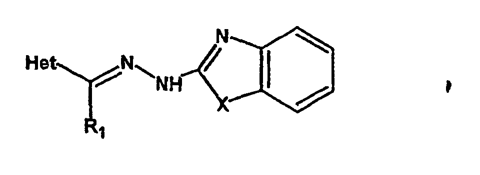 wherein Het= 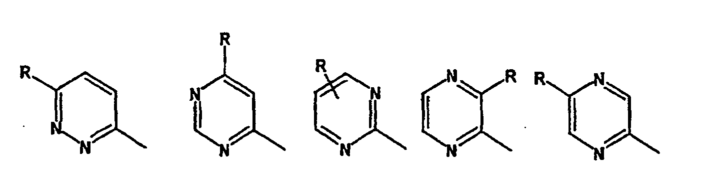 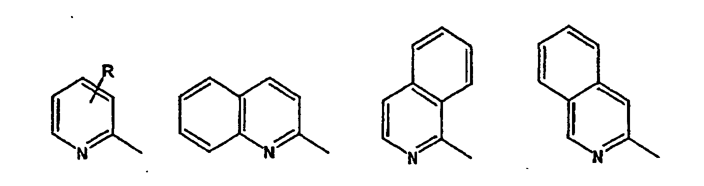 and wherein R = H, CH₃, OCH₃, OH, Cl, Br, F, CF₃, NO₂, NH₂, NHCOCH₃, N(CH₃)₂, phenyl, CN, C=NH(NH₂), C=S(NH₂), C=NH(NHOH), COOH or COOR₄, wherein R₄ = an aliphatic residue or a phenyl group, or CONR₅R₆, wherein R₅ and R₆ are H, an aliphatic substituent or a phenyl group,
R₁ is H, methyl, ethyl, propyl, *iso*-propyl, butyl, *tert*.-butyl, cyclopropyl, cyclohexyl, phenyl, benzyl, or 2-pyridyl, and X = O, as well as the pharmaceutically acceptable salts thereof, to be used as a medicament.

8. A compound as defined in claim 7 to be used as an anti-cancer therapeutic agent.

9. A compound as defined in claim 7 to be used as an anti-tumor therapeutic agent.

10. The use of a compound as defined in claim 7 in the preparation of a medicament for the treatment of cancer of the colon, breast, ovaries or uterus.

## Revendications

1. Composé de formule générale : 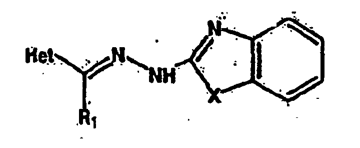 dans laquelle Het = 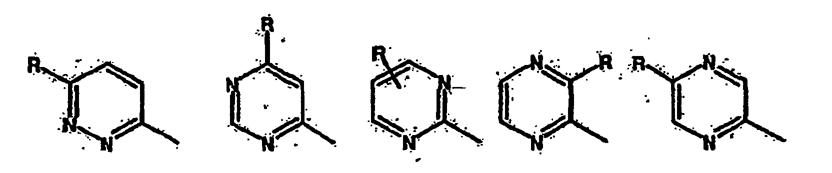 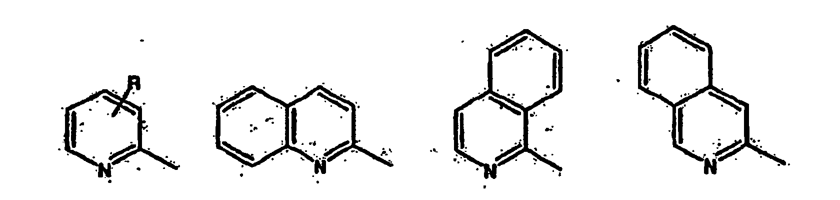 et où R = H, CH₃, OCH₃, OH, Cl, Br, F, CF₃, NO₂, NH₂, NHCOCH₃, N(CH₃)₂, phényle, CN, C=NH(NH₂), C=S(NH₂), C=NH(NHOH), COOH ou COOR₄, où R₄ = radical aliphatique ou groupement phényle, ou CONR₅R₆, R₅ et R₆ représentant H, un substituant aliphatique ou un groupement phényle,
R₁ = H, méthyle, éthyle, propyle, iso-propyle, butyle, tert-butyle, cyclopropyle, cyclohexyle, phényle, benzyle ou 2-pyridyle et
X = O, à condition que si Het = 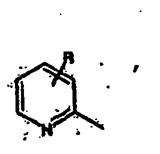 où R est H, R₁ ne représente pas un groupement méthyle, ainsi que ses sels pharmaceutiquement acceptables.

2. Composé selon la revendication 1, soit : 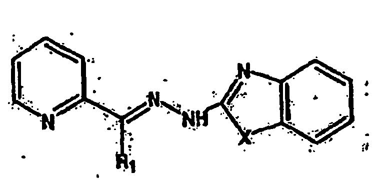 dans lequel R₁ = H, méthyle, éthyle, propyle, iso-propyle, butyle, tert-butyle, cyclopropyle, cyclohexyle, phényle, benzyle ou 2-pyridyle et X = O, à condition que
R₁ ne représente pas un groupe méthyle, ainsi que ses sels pharmaceutiquement acceptables.

3. Composé selon la revendication 1, soit : 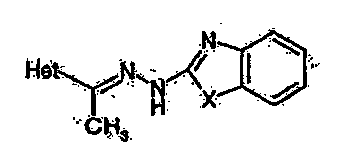 dans lequel Het = 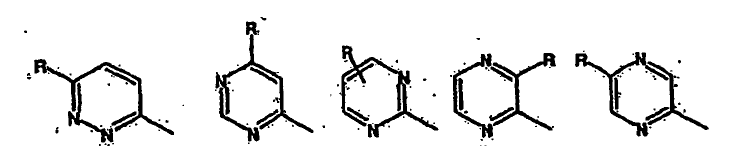 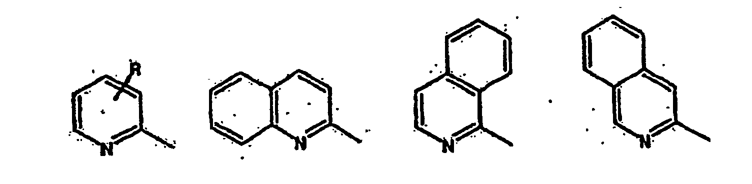
R = H ou CH₃, X = 0,
à condition que, si Het = 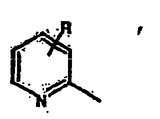 R ne représente pas H,
ainsi que ses sels pharmaceutiquement acceptables.

4. Procédé de fabrication d'un composé de formule générale : 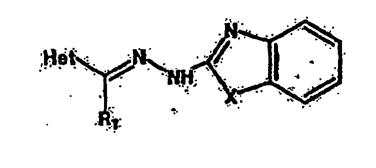 dans laquelle Het = 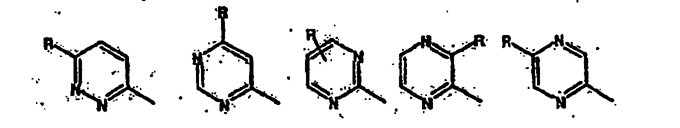 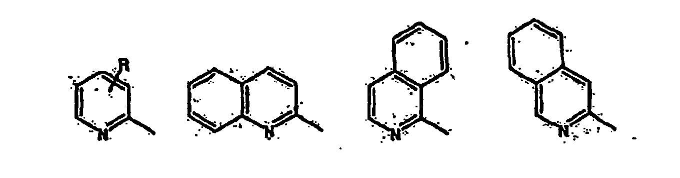 et où R = H, CH₃, OCH₃, OH, Cl, Br, F, CF₃, NO₂, NH₂, NHCOCH₃, N (CH₃)₂, phényle, CN, C=NH (NH₂), C=S (NH₂), C=NH(NHOH), COOH ou COOR₄, où R₄ = radical aliphatique ou groupement phényle, ou CONR₅R₆, R₅ et R₆ représentant H, un substituant aliphatique ou un groupement phényle,
R₁ = H, méthyle, éthyle, propyle, iso-propyle, butyle, tert-butyle, cyclopropyle, cyclohexyle, phényle, benzyle ou 2-pyridyle et X = O, **caractérisé en ce qu'**une cétone de formule générale (III) : 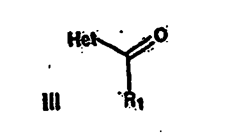
dans laquelle Het et R₁ ont la signification indiquée ci-dessus, est laissé réagi avec une hydrazine de formule générale (II) : 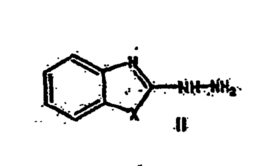 dans laquelle X a la signification indiquée ci-dessus.

5. Procédé selon la revendication 4, **caractérisé en ce qu'**on effectue la réaction dans du méthanol ou de l'éthanol.

6. Procédé selon l'une quelconque des revendications 4 ou 5, **caractérisé en ce que** l'on effectue la réaction en présence d'une quantité catalytique d'un acide approprié, tel que l'acide acétique, l'acide chlorhydrique ou l'acide sulfurique.

7. Composé de formule générale : 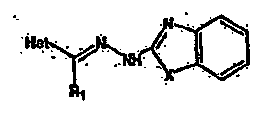 dans laquelle Het = 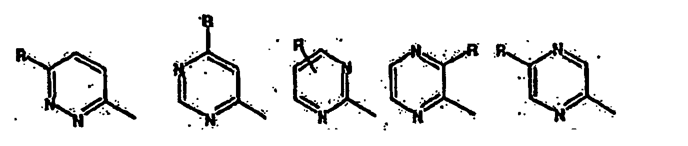 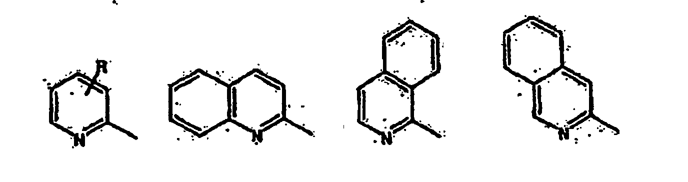 et où R = H, CH₃, OCH₃, OH, Cl, Br, F, CF₃, NO₂, NH₂, NHCOCH₃, N (CH₃)₂, phényle, CN, C=NH (NH₂), C=S(NH₂), C=NH(NHOH), COOH ou COOR₄, où R₄ = radical aliphatique ou groupement phényle, ou CONR₅R₆, R₅ et R₆ représentant H, un substituant aliphatique ou un groupement phényle,
R₁ = H, méthyle, éthyle, propyle, iso-propyle, butyle, tert-butyle, cyclopropyle, cyclohexyle, phényle, benzyle ou 2-pyridyle et X = O,
ainsi que leurs sels pharmaceutiquement acceptables pour utilisation comme médicament.

8. Composé tel que défini dans la revendication 7 pour utilisation comme remède anti-cancéreux.

9. Composé tel que défini dans la revendication 7 pour utilisation comme remède anti-tumeur.

10. Utilisation d'un composé, tel que défini dans la revendication 7, pour la fabrication d'un médicament pour le traitement du cancer du gros intestin, du cancer du sein, du cancer des ovaires ou du cancer de l'utérus.
